# EUROPEAN PATENT SPECIFICATION

(11) **EP 2 341 900 B1**
(45) Date of publication and mention of the grant of the patent: **20.02.2019**
(21) Application number: 09820803.6
(22) Date of filing: 12.10.2009
(51) Int. Cl.: A61K 31/192, A61K 31/167, A61P 19/00, A61P 29/00

(54) **A MEDICINAL PRODUCT AND TREATMENT**
MEDIZINISCHES PRODUKT UND BEHANDLUNGSVERFAHREN DAMIT
PRODUIT MÉDICAMENTEUX ET TRAITEMENT ASSOCIÉ

(30) Priority: 14.10.2008 NZ 56961208
(43) Date of publication of application: 13.07.2011
(73) Proprietor: AFT Pharmaceuticals Limited, Auckland 0622 (NZ)
(72) Inventor: ATKINSON, HARTLEY CAMPBELL, Auckland 0632 (NZ)
(74) Representative: Franks & Co (South) Limited
(86) International application number: PCT/NZ2009/000220
(87) International publication number: WO 2010/044681

(56) References cited:
- EP-A1- 0 109 281
- WO-A1-2007/034135
- WO-A2-2006/004449
- AU-B2- 605 538
- CA-C- 1 336 687
- US-A1- 2009 264 530
- C. JANE NIKLES ET AL.: 'Do Individualized Medication Effectiveness Tests (N-of-1 trials) Change Clinical Decisions About Which Drugs to Use for Osteoarthritis and Chronic Pain' AMERICAN JOURNAL OF THERAPEUTICS vol. 12, 2005, pages 92 - 97, XP008136647

## Description

### TECHNICAL FIELD

The invention relates to a medicament comprising paracetamol and ibuprofen for the treatment of osteoarthritis or rheumatoid arthritis.

### BACKGROUND

Tablets having a combination of paracetamol (about 475 mg to about 500 mg) and ibuprofen (about 125 mg to about 150 mg) are known from published patent specification WO 2006/004449 by AFT Pharmaceuticals. That WO specification discloses the use of such combination for reducing pain after dental surgery.

The process of producing a tablet comprising paracetamol (500mg) and a non-steroidal anti-inflammatory drug (NSAID) such as ibuprofen (200mg) is also known and disclosed in WO2007/034135 A1.

It has now been discovered that combinations of paracetamol and ibuprofen are sufficient to give surprising synergistic results when used for reducing discomfort associated with osteoarthritis and rheumatoid arthritis.

Osteoarthritis may involve inflammation of joints resulting from abnormal wear of the cartilage which serves to cushion a joint, coupled with a decrease in the level of the synovial fluid which lubricates the joint. As bone surfaces at the joint become progressively less protected the patient suffers pain when the affected joints are made to bear normal body weight, for example when standing or walking. This can develop to the stage where muscles suffer atrophy and ligaments become lax. Rheumatoid arthritis is an autoimmune disorder which can involve the immune system attacking one's joints, also causing inflammation. It can be a disabling and painful condition leading to loss of mobility due to pain.

Paracetamol at 4,000 mg/day, taken in four doses of 1,000 mg each, is considered sufficient for low level pain or analgesic relief but for many patients it is less than effective for producing the sort of relief required for moderate to severe osteoarthritis or moderate to severe rheumatoid arthritis. It is known to treat moderate to severe osteoarthritis with ibuprofen at up to 2,400 mg per day (ie in three doses of 800 mg each). Intake of ibuprofen at that level is considered to be anti-inflammatory treatment rather than merely analgesic treatment (for analgesic treatment with ibuprofen a patient would normally only take up to 1,200 mg/day in three doses of 400 mg each). However, anti-inflammatory treatment with ibuprofen at 2,400 mg/day from three doses can result in undesirable side affects, for example adverse cardio renal conditions, thrombotic risks and gastrointestinal bleeding. Reducing the daily dose of ibuprofen reduces the risk of such side affects but at the same time gives substantially less pain and/or anti-inflammatory relief.
For many patients suffering from osteoarthritis or rheumatoid arthritis, combined ibuprofen and paracetamol treatment significantly reduces the daily dose of ibuprofen which would otherwise be necessary for achieving suitable relief. Thus the risk of side affects normally attributable to high levels of ibuprofen can be substantially reduced for some patients without at the same time compromising patient comfort, at least to any significant extent.

### SUMMARY OF THE INVENTION

According to one aspect of the invention there is provided a medicament for use in treating moderate or severe osteoarthritis or moderate or severe rheumatoid arthritis, wherein the medicament comprises a combination composition comprising ibuprofen and paracetamol to be administered in doses of 250 mg to 300 mg ibuprofen and 950 mg to 1,000 mg paracetamol per dosage event.
Optionally the composition is in dosage units each comprising 125 mg to 150 mg ibuprofen and 475 mg to 500 mg paracetamol.

The medicament is for use in treating moderate or severe osteoarthritis.

The medicament is for use in treating moderate or severe rheumatoid arthritis.

Optionally the composition comprises dosage units having 150 mg ibuprofen and 500 mg paracetamol.
Optionally the medicament is for taking in two dosage units up to four times each day.
Optionally the medicament is for taking In two dosage units four times each day.
Optionally the dosage units are tablets or capsules.
Optionally the composition is to be administered in doses of 300 mg ibuprofen and 1,000 mg paracetamol per dose.
Optionally the composition is in liquid form.

### BRIEF DESCRIPTION OF DRAWINGS

Some preferred embodiments of the invention will now be described by way of example and with reference to the accompanying drawings, of which:
- **Figure 1**: illustrates the efficacy of a preferred form of the present invention in relation to WOMAC pain scores; and
- **Figure 2**: further illustrates the efficacy of a preferred form of the invention in relation to Global Pain Rating scores.

### DETAILED DESCRIPTION

In a preferred form of the invention there is a tablet which has 125 mg to 150 mg ibuprofen and 475 mg to 500 mg paracetamol in combination. Most preferably the ibuprofen and paracetamol content of the tablet is 150 mg and 500 mg respectively. By taking two tablets every 6 hours a patient can receive a total of 1,200 mg ibuprofen and 4,000 mg paracetamol over a 24 hour period. Alternatively the tablet can be double strength so that only one tablet is required to deliver 250 mg to 300 mg ibuprofen and 950 mg to 1,000 mg paracetamol in combination.

The level of relief obtained from two tablets taken up to four times each day is adequate for at least some patients suffering from osteoarthritis or rheumatoid arthritis, particularly the moderate to severe forms of those conditions. This is surprising because moderate to severe osteoarthritis and rheumatoid arthritis are inflammatory conditions and the level of ibuprofen and paracetamol delivered by two tablets at each dose would not be expected to significantly alleviate the discomfort caused by such inflammation. However when ibuprofen and paracetamol are combined at levels of 250-300 mg and 950-1,000 mg respectively (eg from one or two tablets) a significant benefit is achieved, particularly if such treatment is repeated at 6 hourly intervals. With at least osteoarthritis there is an expectation that significantly greater levels of ibuprofen are required, for example 800 mg taken three times over a 24 hour period (ie 2,400 mg/day) for the treatment of at least moderate to severe cases. The combination therapy described herein is sufficient to deliver relief to at least some patients suffering from mild, moderate or severe osteoarthritis, and is particularly helpful to some patients in cases of moderate to severe osteoarthritis.

### Clinical Study

To exemplify the efficacy of a preferred embodiment of the invention a prospective, randomised, double-blind study was run to measure its effect on human patients suffering from osteoarthritis. Four groups of patients were selected and each group was given one or other of the following medications over a four week period:

| **Group name** | **Medication taken** | **Total amount of medication ingested per day** |
|---|---|---|
| Paracetamol | 2 x 500 mg paracetamol tablets four times daily | 4,000 mg |
| Ibuprofen low | 2 x 150 mg ibuprofen tablets four times daily | 1,200 mg |
| Ibuprofen high | 2 x 300 mg ibuprofen tablets four times daily | 2,400 mg |
| combination | 2 x combination tablets four times daily, each tablet having 500 mg paracetamol plus 150 mg ibuprofen | 4,000 mg paracetamol plus 1,200 mg ibuprofen |

All patients were aged from 45-80 and had been suffering from chronic knee pain due to osteoarthritis for at least 6 months. Patients went through a washout period of their existing osteoarthritis treatments. The first three groups each had 8 patients and the fourth group started with 9 patients. One of the patients in the fourth group did not complete the study. At the beginning of the study, and also at conclusion of each subsequent week, patients were required to visit a physician and complete a WOMAC questionnaire and a Global Pain Rating Assessment.

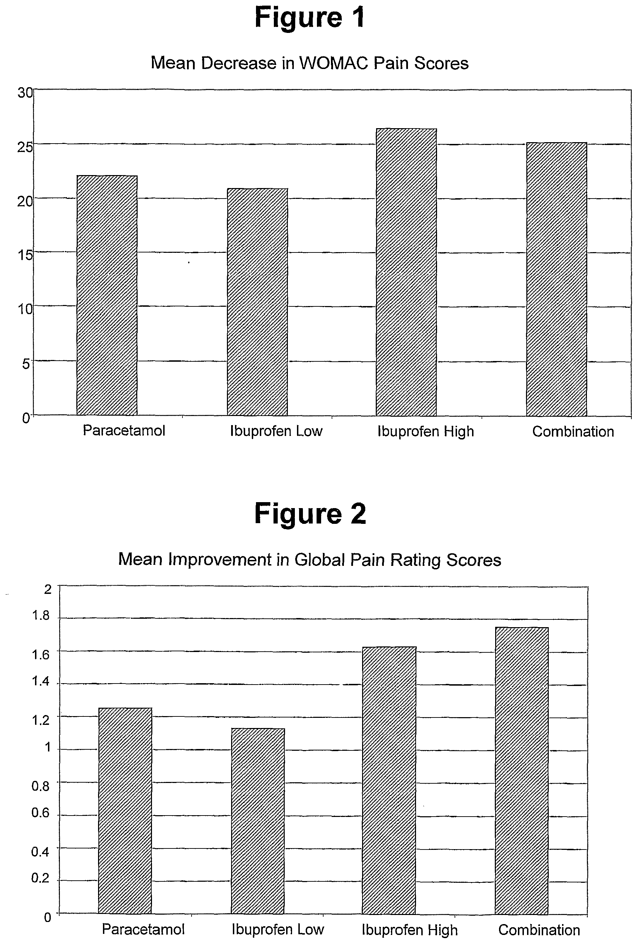

The WOMAC questionnaire involved a series of questions related to the level of pain suffered by the patients. Patients were required to mark their answers to each question separately, in a quantitative manner, using a 100 mm long analogue scale. The difference in scores between the start and end of the study was compared. The mean decrease in WOMAC pain scores is shown graphically in figure 1. It can be seen there that patients in the Combination group achieved significantly better pain relief than those in the Paracetamol and Ibuprofen Low groups. Indeed patients in the Combination group received virtually equivalent relief to those in the Ibuprofen High group, but without the same risks of undesirable side effects.

The Global Pain Rating Assessment involved four rating categories, namely:

| | |
|---|---|
| • Nil | (no pain noticed); |
| • Mild | (pain noticed but no disruption to normal daily activity); |
| • Moderate | (pain noticed sufficient to reduce or affect daily activity); |
| • Severe | (inability to work or perform daily activity). |

Patients rated their level of pain according to one or other of these categories at the start of the study, and also at the end of each subsequent week. The improvement in patient scores was assessed. For example patients who began with a "moderate" pain rating, and then at the end of the study had only a "mild" pain rating, improved by 1 rating group. By way of further example, patients who began with a severe pain rating, and then ended the study with a "mild" pain rating, improved by 2 rating groups. The mean improvement in patients in each group in terms of the number of rating groups was assessed and graphed as shown in figure 2. The graph shows that patients in the Combination group received significantly superior pain relief to those in the Paracetamol and Ibuprofen Low groups. It also shows that patients in the Combination group received better pain relief to those in Ibuprofen High group but, again, without the same risk of adverse side affects.
In terms of adverse side effects patients were asked to keep a record of these, if any. There were no confirmed or probable adverse side effects recorded for the Combination group, for the Paracetamol group or for Ibuprofen Low group. However in the Ibuprofen High group two probable adverse effects were recorded, both related to gastric discomfort.
It is surprising that the graphs of figures 1 and 2 show the Combination providing substantially equivalent, or better, pain relief when compared to Ibuprofen High, particularly given that the Combination only involved half the amount of Ibuprofen. It is also surprising that the graphs show the Combination providing significantly better pain relief than Ibuprofen Low because both medications involved the same amount of ibuprofen and because the paracetamol in the Combination would not be expected to contribute relief to patients already taking 1,200 mg per day ibuprofen.
While effective relief for discomfort from osteoarthritis may be achieved within the first dose interval (2 tablets as described above), it is preferred to continue this with a quarterly administration regime. Two tablets or capsules four times a day is a relatively easy regime to be met by a user. Increasing from this amount may result in dosage and administration problems. This is an additional advantage over and above the potential for reduction in adverse side effects.
The tablets or capsules referred to above can be prepared and presented in the same way described in published patent specification WO 2006/004449 by AFT Pharmaceuticals.

### Alternative Chemical Forms

While ibuprofen and paracetamol have been specifically referred to in this specification, suitable other pharmaceutically acceptable forms of the two actives (eg salts, etc) may also be used and are intended to be embraced by references to the actives per se, with the weight amounts adjusted accordingly. For example, when a salt form is used in the formulation sufficient quantity will need to be included to meet the desired amount of acid (e.g., 342 mg ibuprofen lysinate corresponds with 200 mg ibuprofen). Thus, for example, a reference to 150 mg ibuprofen may be construed as a reference to the therapeutically equivalent amount of ibuprofen lysinate.

While some preferred embodiments of the invention have been described by way of example it should be appreciated that modifications and improvements can occur without departing from the scope of the appended claims.

## Claims

1. A medicament for use in treating moderate or severe osteoarthritis or moderate or severe rheumatoid arthritis, wherein the medicament comprises a combination composition comprising ibuprofen and paracetamol to be administered in doses of 250 mg to 300 mg ibuprofen and 950 mg to 1,000 mg paracetamol per dosage event.

2. A medicament for use according to claim 1, wherein the composition is in dosage units each comprising 125 mg to 150 mg ibuprofen and 475 mg to 500 mg paracetamol

3. A medicament for use according to claim 1 or claim 2, wherein the medicament is for use in treating moderate or severe osteoarthritis.

4. A medicament for use according to claim 1 or claim 2, wherein the medicament is for use in treating moderate or severe rheumatoid arthritis.

5. A medicament for use according to claim 4, wherein the composition comprises dosage units having 150 mg ibuprofen and 500 mg paracetamol.

6. A medicament for use according to any one of the preceding claims, wherein the medicament is for taking in two dosage units up to four times each day.

7. A medicament for use according to any one of the preceding claims, wherein the medicament is for taking In two dosage units four times each day.

8. A medicament for use according to claim 5 or claim 6, wherein the dosage units are tablets or capsules.

9. A medicament for use according to any one of the preceding claims, wherein the composition is to be administered in doses of 300 mg ibuprofen and 1,000 mg paracetamol per dose.

10. A medicament for use according to any one of claims 1 to 7, wherein the composition is in liquid form.

## Patentansprüche

1. Medikament zur Verwendung bei der Behandlung mäßiger oder schwerer Arthrose oder mäßiger oder schwerer rheumatoider Arthritis, wobei das Medikament eine kombinierte Zusammensetzung aus ibuprofen und Paracetamol umfasst, die in Dosen von 250 mg bis 300 mg Ibuprofen und 950 mg bis 1000 mg Paracetamol pro Dosisgabe zu verabreichen ist.

2. Medikament zur Verwendung nach Anspruch 1, wobei die Zusammensetzung in Dosiereinheiten von jeweils 125 mg bis 150 mg ibuprofen und 475 mg bis 500 mg Paracetamol vorliegt.

3. Medikament zur Verwendung nach Anspruch 1 oder Anspruch 2, wobei das Medikament zur Behandlung mäßiger oder schwerer Arthrose dient.

4. Medikament zur Verwendung nach Anspruch 1 oder Anspruch 2, wobei das Medikament zur Behandlung mäßiger oder schwerer rheumatoider Arthritis dient.

5. Medikament zur Verwendung nach Anspruch 4, wobei die Zusammensetzung Dosiereinheiten von 150 mg Ibuprofen und 500 mg Paracetamol umfasst.

6. Medikament zur Verwendung nach einem der vorhergehenden Ansprüche, wobei das Medikament zur Einnahme in zwei Dosiereinheiten bis zu vier Mal täglich dient.

7. Medikament zur Verwendung nach einem der vorhergehenden Ansprüche, wobei das Medikament zur Einnahme in zwei Dosiereinheiten vier Mal täglich dient.

8. Medikament zur Verwendung nach Anspruch 5 oder Anspruch 6, wobei die Dosiereinheiten Tabletten oder Kapseln sind.

9. Medikament zur Verwendung nach einem der vorhergehenden Ansprüche, wobei die Zusammensetzung in Dosen von 300 mg Ibuprofen und 1000 mg Paracetamol pro Dosis zu verabreichen ist.

10. Medikament zur Verwendung nach einem der Ansprüche 1 bis 7, wobei die Zusammensetzung in flüssiger Form vorliegt.

## Revendications

1. Médicament destiné à être utilisé dans le traitement de l'ostéoarthrite modérée ou sévère ou de la polyarthrite rhumatoïde modérée ou sévère, le médicament comprenant une composition combinée comprenant de l'ibuprofène et du paracétamol à administrer dans des doses de 250 mg à 300 mg d'ibuprofène et de 950 mg à 1 000 mg de paracétamol par événement de dosage.

2. Médicament destiné à être utilisé selon la revendication 1, dans lequel la composition est contenue dans des unités posologiques comprenant chacune de 125 mg à 150 mg d'ibuprofène et de 475 mg à 500 mg de paracétamol

3. Médicament destiné à être utilisé selon la revendication 1 ou la revendication 2, le médicament étant destiné à être utilisé dans le traitement de l'ostéoarthrite modérée ou sévère.

4. Médicament destiné à être utilisé selon la revendication 1 ou la revendication 2, le médicament étant destiné à être utilisé dans le traitement de la polyarthrite rhumatoïde modérée ou sévère.

5. Médicament destiné à être utilisé selon la revendication 4, dans lequel la composition comprend des unités posologiques contenant 150 mg d'ibuprofène et 500 mg de paracétamol.

6. Médicament destiné à être utilisé selon l'une quelconque des revendications précédentes, le médicament devant être pris en deux unités posologiques jusqu'à quatre fois par jour.

7. Médicament destiné à être utilisé selon l'une quelconque des revendications précédentes, le médicament devant être pris en deux unités posologiques quatre fois par jour.

8. Médicament destiné à être utilisé selon la revendication 5 ou la revendication 6, dans lequel les unités posologiques sont des comprimés ou des capsules.

9. Médicament destiné à être utilisé selon l'une quelconque des revendications précédentes, dans lequel la composition doit être administrée en doses de 300 mg d'ibuprofène et de 1 000 mg de paracétamol par dose.

10. Médicament destiné à être utilisé selon l'une quelconque des revendications 1 à 7, dans lequel la composition est sous forme liquide.
